# EUROPEAN PATENT APPLICATION

(11) **EP 3 842 803 A1**
(43) Date of publication of application: **30.06.2021**
(21) Application number: 19219456.1
(22) Date of filing: 23.12.2019
(51) Int. Cl.: G01N 33/68

(54) **CXCL14 IN POLYCYSTIC OVARY SYNDROME**

(71) Applicant: Katholieke Universiteit Leuven, 3000 Leuven (BE); Hospital Sant Joan de Deu, 08950 Esplugues de Llobregat Barcelona (ES)
(72) Inventor: IBÁÑEZ, Lourdes, 08022 Barcelona (ES); DE ZEGHER, Francis, 3080 Tervuren (BE)

(57) **Abstract**

The invention relates to method of measuring CXCL14 in a serum sample, in the diagnosis of PCOS in an adolescent girl.

## Description

### Field of the invention

The invention relates to the diagnosis of polycystic ovary syndrome.

### Background of the invention

Adipose tissue plasticity is growingly recognized as a relevant factor for the development of metabolic syndrome, independently of the presence or absence of obesity. The acquisition of a "brown" or "beige" phenotype by adipose tissue is considered protective against hyperglycemia and hyperlipidemia and the relative protection against these alterations in young-versus-elder individuals is associated with the known prevalence of the brown/beige phenotype in early human development (1). CXCL14 (C-X-C motif chemokine ligand-14) is a chemokine produced by active brown/beige adipose tissue, capable of improving glucose metabolism in insulin resistant rodent models (2).

Central (hepato-visceral) fat excess and insulin resistance are common metabolic co-morbidities in girls and young women with polycystic ovary syndrome (PCOS) (3,4). Treatment with a low-dose combination of one mixed anti-androgen and anti-mineralocorticoid (spironolactone), and two insulins sensitizers (pioglitazone plus metformin) (SPIOMET) has been shown to improve the metabolic condition of these patients to a better extent than oral contraception (OC) (5). However, the specific cellular and tissue targeting, and the relative role of each of the SPIOMET components in the context of PCOS is poorly understood. It has been recently reported that women with PCOS have lower brown adipose tissue (BAT) activity as compared to healthy controls (6,7) and experimental data suggest that BAT activation might be a promising therapeutic option for PCOS (8). Chazenbalk et al. (2012) Clin Endocrinol Metab 97(5), E765-E770 disclose expression levels of CXCL14 in PCOS individuals.

### Summmary of the invention

CXCL14 (C-X-C motif chemokine ligand-14) is a chemokine recently found to be released by active brown fat and to be protective against insulin resistance in experimental models. Polycystic ovary syndrome (PCOS) in adolescent girls is usually related to central (hepato-visceral) fat excess and insulin resistance, and associates to co-morbidites including type 2 diabetes. Treatment with a low-dose combination of one mixed anti-androgen and anti-mineralocorticoid (spironolactone), and two insulin sensitizers (pioglitazone/metformin) (SPIOMET), is particularly effective in improving these metabolic derangements. Adipose tissue may be involved in the metabolic alterations of PCOS and it is a likely target site of therapeutic action. The present invention discloses that serum CXCL14 is abnormally reduced in PCOS patients, and that this reduction correlates positively with lowered high-molecular-weight adiponectin levels. One-year SPIOMET treatment normalized CXCL14 concentrations and improved the metabolic status. Pioglitazone induced CXCL14 expression in differentiating human adipocytes, in parallel with the induction of marker genes of brown adipogenesis, whereas spironolactone induced CXCL14 expression and release in differentiated human adipocytes. In conclusion, insulin sensitization with SPIOMET normalizes CXCL14 levels in girls with PCOS, indicating that this combination may act through promoting a brown-like phenotype in adipose tissue, potentially improving glucose homeostasis and possibly reducing diabetes risk. Treatment with a low-dose combination of one mixed anti-androgen and anti-mineralocorticoid (spironolactone), and two insulin sensitizers (pioglitazone/metformin) (SPIOMET) is particularly effective in improving the endocrine-metabolic derangements in adolescent girls with polycystic ovary syndrome (PCOS).
CXCL14 (C-X-C motif chemokine ligand-14) is a chemokine released by active brown fat and protective against insulin resistance in experimental models.
Serum CXCL14 levels are abnormally reduced in PCOS patients.
SPIOMET treatment for one year normalized CXCL14 concentrations and improved the endocrine-metabolic status of PCOS patients.
Pioglitazone and spironolactone, drug components of SPIOMET, induce CXCL14 expression and release in human adipocytes, in parallel with the induction of marker genes of brown adipogenesis.
CXCL14 is described herein as a novel biomarker for PCOS. In addition, CXCL14 can function as a potential mediator of the beneficial effects of the SPIOMET combination and may hold the capacity of serving as therapeutic modulator of the disorder.

The present invention described the abnormally low levels of CXCL14 in girls with PCOS, the differential effects of SPIOMET and OC on circulating CXCL14 in relation to metabolic improvement, and the effects of SPIOMET components on the expression and release of CXCL14 in a cellular model of human adipocytes.

The invention is summarised in the following statements:
1. In vitro use of reagents for measuring CXCL14 in a serum sample, in the diagnosis of PCOS in an adolescent girl.
2. The use according to statement 1, wherein the adolescent girl is non-obese.
3. In vitro use of reagents for measuring CXCL14 in serum in monitoring the efficacy of a PCOS treatment in an adolescent girl.
4. The use according to statement 3, wherein the PCOS treatment is a treatment with with spironolactone, pioglitazone and metformin (SPIOMET).
5. The use according to statement 3 or 4, wherein the adolescent girl is non-obese.
6. The use according to any one of statements 3 to 5, wherein the monitoring identifies responders or non responders to said treatment.
7. The use according to anyone of statements 3 to 8, wherein an increase of the concentation of CXCL14, compared to the concentation prior to the treatment is indicative of the patient being a responder.
8. The use according to any one of statements 1 to 7 wherein the reagents are oligonucleotides for the detection of CXCL14 mRNA.
9. The use according to any one of statements 1 to 7, wherein the reagents are binding agents specifically binding to CXCL14 protein.
10. In vitro method of diagnosing PCOS in an adolescent girl, comprising the steps of:
   determining in a serum sample of said adolescent girl, the concentration of CXCL14, comparing the determined concentation with a reference value of a healthy adolescent girl, determining from said comparison, whether said adolescent girl is a PCOS patient.
11. The method according to statement 10, wherein a low concentation of CXCL14 in the sample compared to said reference value is indicative for said adolescent girl being a PCOS patient.
12. In vitro method for monitoring the efficacy of a PCOS treatment in an adolescent girl, comprising the steps of:
   determining in a serum sample of said adolescent girl prior to or at the onset of said treatment, a first concentration of CXCL14,
   determining during the treatment in a serum sample of said adolescent girl, a second concentration of CXCL14,
   comparing the first and the second concentratiob, wherein an increase of the concentration, compared to the first concentration is indicative of the adolescent girl being a reponder to said treatment.
13. The method according to statement 12, wherein the treatment is a treatment with spironolactone, pioglitazone and metformin (SPIOMET).
14. The method according to statement 12 or 13, wherein the step of determining the second concentration is performed between 1 and 12 months after the onset of said treatment.

### DETAILED DESCRIPTION

### Figure Legends

**Figure 1**
   A. Baseline circulating CXCL14 concentrations in girls with polycystic ovary syndrome (PCOS, N= 52) and in healthy age-matched controls (N= 21). Data are mean + SEM.
   B. Longitudinal results of CXCL14 serum concentrations in girls with PCOS who received an oral contraceptive (OC, white circles, N= 16) or low-dose spironolactone, pioglitazone and metformin (SPIOMET, black circles, N= 15). The dotted line is the mean in healthy controls (N=21); the shaded area represents the ± SEM in those healthy controls.
   *P <0.01, patients vs controls at baseline; on-treatment differences between controls and the OC subgroup; ^{#}P <0.01, for changes 0-1 year in the SPIOMET subgroup; ^{&}P <0.05 for changes 0-1 year between the OC subgroup and the SPIOMET subgroup.

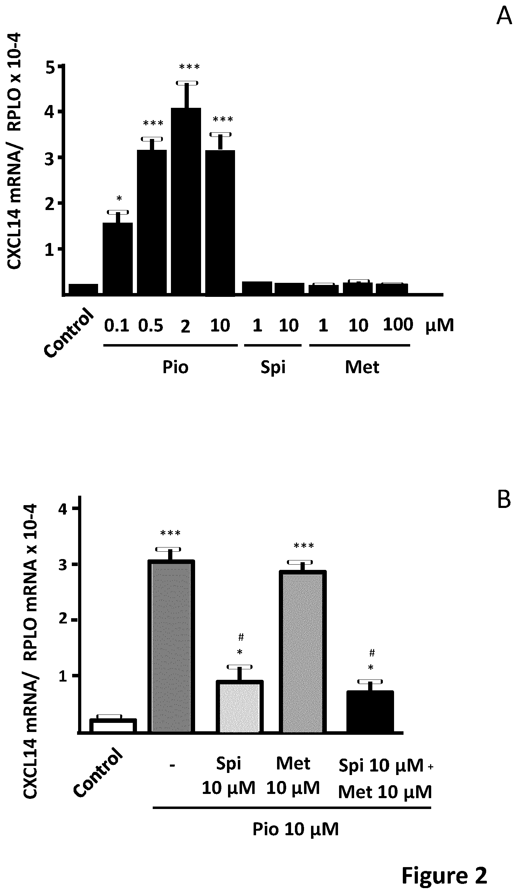

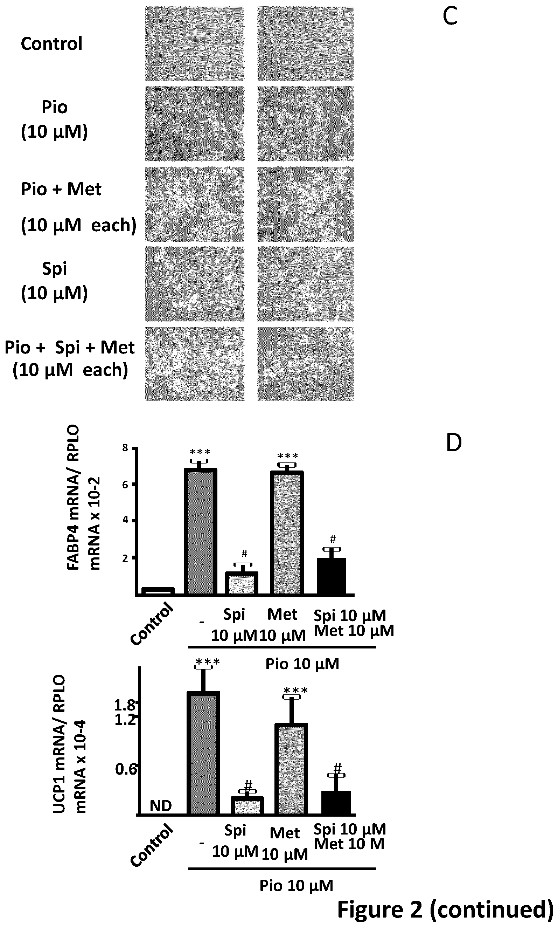

**Figure 2** Effects of pioglitazone (Pio), spironolactone (Spi) and metformin (Met) on adipogenic differentiation of SHBS human pre-adipocytes in culture. SGBS human pre-adipocytes were treated chronically (10 days) with pioglitazone, spironolactone or metformin alone (A) or in combination (B, C, D) at the indicated doses across the adipoipogeic differentiation process.
   A, B. CXCL14 transcript levels are presented as means + SEM from 4-5 independent experiments, and are expressed relative to values from untreated control cells.
   C. Representative photomicrographs of adipocyte cell cultures differentiating in the presence of the indicated components.
   D. FABP4 and UCP1 transcript levels are presented as means + SEM from 3-4 independent experiments, and are expressed relative to values from untreated control cells.
   *P <0.05, **P <0.01, and ***P <0.001 for each component vs control; ^{#}P < 0.05 relative to pioglitazone alone. ND; non detected.
**Figure 3** Effects of pioglitazone (Pio), spironolactone (Spi) and metformin (Met) on CXCL14 gene expression and CXCL14 protein release in human adipocytes. SGBS cells were treated acutely (24h, at the indicated doses) when already differentiated adipocytes. CXCL14 transcript level (A) and CXCL14 protein levels in cell culture medium (B) are presented as means ± SEM from 4-5 independent experiments, and are expressed relative to values from untreated control cells.
   *P <0.05, **P <0.01, and ***P <0.001 for each component vs control
**Figure 4** show a review of clinical trials (A: trial 1; B: trial 2) 1as discussed in the examples section.

***"CXCL14"*** refers to "C-X-C motif chemokine ligand 14" [Gene ID: 9547]. Alternative names wich are found in the literature are KEC, KS1, BMAC, BRAK, NJAC, MIP2G, MIP-2g and SCYB14.
**"Polycystic ovary syndrome (PCOS)"** is a prevalent disorder in adolescent girls, commonly driven by hepato-visceral fat excess, usually presenting with hirsutism and menstrual irregularity, and often followed by subfertility and type 2 diabetes.
Criteria for diagnosis of PCOS in adolescent girl are described in detail in [Ibanez et al. (2017) Horm Res Pediatr 88, 371-395] and include irregular menses or oligomenorrhea, biochemical and/or clinical evidence of hyperandrogenism, and optionally polycystic ovarian morphology and severe cystic acne. PCOS is a common endocrine disorder among women of reproductive age. In humans, a woman's fertility peaks in the early and mid-20s, after which it starts to decline slowly, with a more dramatic drop at around 35. Menopause, or the cessation of menstrual periods, generally occurs in the 40s and 50s and marks the cessation of fertility. Therefore, within the context of the present invention childbearing age preferably refers to an age between 20 and 45 years old, more preferably to an age between 20 and 35 years old. Oligo-ovulatory androgen excess in adolescent girls or women (a further term that can be used to refer to polycystic ovary syndrome [PCOS] according to NIH) is a major cause of subfertility and relates to hepatic steatosis, independently of obesity. PCOS may result in enlarged ovaries, and physically manifest as excess hair growth, acne, obesity or infrequent, prolonged menstrual cycles, hirsutism, seborrhoea and menstrual irregularity. It is unknown whether early interventions influence the post-treatment rates of spontaneous ovulations. PCOS is typically diagnosed via one of two methods: clinical or biochemical hyperandrogenism. Clinical hyperandrogenism is defined as moderate to severe hirsutism with a Ferriman-Gallway score of > 9 AND a free testosterone level that is equal to or > 50% above the ULN(upper limit of normal), while biochemical hyperandrogenism is defined as free testosterone level that is equal to or > 75% above the ULN.
The term **"contraceptive"** refers to the following. There are two main types of hormonal contraceptive formulations: combined methods which contain both an oestrogen and a progestin, and progestogen-only methods which contain only progesterone or one of its synthetic analogues (progestins). The combined oral contraceptive pill (COCP), often referred to as the birth control pill or colloquially as "the pill", is a type of birth control that is designed to be taken orally by women. It includes a combination of an oestrogen (usually ethinylestradiol) and a progestogen (specifically a progestin). When taken correctly, it alters the menstrual cycle to eliminate ovulation and prevent pregnancy.
**"Non-alcoholic fatty liver disease"** (NAFLD) refers to a disorder characterized by an abnormal accumulation of fat in the liver, due to causes other than excessive alcohol use. NAFLD is a continuum of liver abnormalities, from non-alcoholic fatty liver (NAFL) to non-alcoholic steatohepatitis (NASH). These diseases begin with fatty accumulation in the liver (hepatic steatosis). A liver can remain fatty without disturbing liver function (NAFL), but by various mechanisms and possible insults to the liver, it may also progress into a non-alcoholic steatohepatitis (NASH), a state in which steatosis is combined with inflammation and sometimes fibrosis (steatohepatitis).
**"Adolescence"** refers to the age range between 10 and 24 years more typically between 10 and 19 years).
**"Non-obese"** refers to a body mass index below 30, typically below 27,5 and more typically below 25.
**"Responder"** refers to a PCOS patient wherein a treatment is accompanied by a rise of the concentrations of CXL14, typically above the -2 SD limit of a reference range. The term **"central fat"** means the hepato-visceral adipose tissue (VAT) and/or ectopic hepato-visceral fat storage. Central fat was defined sum of visceral fat (by MRI, in cm2) and hepatic fat (by MRI, in %).
**"Expression level"** may be determined by measuring the amount of mRNA in the sample fluid using a variety of suitable reagents. The expression level of the mRNA can be determined, for example, with an assay for global gene expression in a biological fluid (e.g. using a microarray assay for mRNA expression profiling analysis, or a ready-to-use mRNA qPCR plate), or by specific detection assays, such as quantitative PCR, quantitative reverse-transcription (real-time) PCR (qRT-PCR), locked nucleic acid (LNA) real-time PCR, or northern blotting. The measurement of the expression level of a microRNA in a biological fluid may be carried out with an oligonucleotide probe specific for the detection of said microRNA. Said oligonucleotide probe may bind directly and specifically to the microRNA, or may specifically reverse transcribe said microRNA. Alternatively, said oligonucleotide probe may bind a cDNA obtained from said microRNA. Said oligonucleotide probe may also amplify a cDNA obtained from said microRNA.
**"kit"** refers to any combination of reagents or apparatus that can be used to perform a method of the invention. Kits for use in the present invention comprise probes for detection of a limited amount of mRNA (up to 10, 15 or 20 mRNA) to distinguish over arrays containing probes for more than 100 or 1000 mRNA. Apart from probes for detecting CXCL14 as recited in the claims the kit may comprise probes for the detect may comprise housekeeping mRNA. The kit may also comprise instructions for use to diagnose whether a subject classifies as a responder to the IL10, PINS, anti-CD3 diabetes therapy.
**"oligonucleotide probe"** refers to a short sequence of nucleotides that match a specific region of a microRNA or a cDNA obtained from said microRNA, or fragments thereof, and then used as a molecular probe to detect said microRNA or cDNA sequence.
An oligonucleotide probe **"specific for the detection of a microRNA"** for example refers to an oligonucleotide probe that bind directly and specifically to a microRNA or a fragment thereof, or specifically reverse transcribe said microRNA. Alternatively, said oligonucleotide probe may bind specifically to a cDNA obtained from said microRNA. Said oligonucleotide probe may also specifically amplify a cDNA obtained from said microRNA.
Alternatively a reference is obtained by using a sample or a pool of samples from a population of validated responders or by using a sample or a pool of samples from a population of validated non-responders. Experimental data can be compared with control data and classified as belonging to the responder or non-responder group. Compared with a control group of non-responders a non-responder will have about equal expression levels, and a responder will have lower expression levels. Equally, compared with a control group of responders a responder will have about equal expression levels, and a non-responder will have higher expression levels.
General techniques useful in microRNA detection are disclosed in "MicroRNA Expression Detection Methods", Wang Zhiguo, Yang Baofeng, 2010, XX; "Circulating MicroRNAs Methods and Protocols", series: Methods in Molecular Biology, Vol. 1024 Ochiya, Takahiro (Ed.) 2013.

Determination of expression values obtained by qPCR can be expressed as 2^-dct values as explained for example in Livak & Schmittgen (2001) Methods 25, 402-408. This value represents the fold change in expression of the target iRNA relative to a control mRNA.
The term **"statistically significant"** differences between the groups studied, relates to condition when using the appropriate statistical analysis (e.g. Chi-square test, t-test) the probability of the groups being the same is less than 5%, e.g. p<0,05. In other words, the probability of obtaining the same results on a completely random basis is less than 5 out of 100 attempts.
Any technique known to one of skill in the art for detecting and measuring RNA expression levels can be used in accordance with the methods described herein. Non-limiting examples of such techniques include microarray analysis, Northern blotting, nuclease protection assays, RNA fingerprinting, polymerase chain reaction, ligase chain reaction, Qbeta replicase, isothermal amplification method, strand displacement amplification, transcription based amplification systems, quantitative nucleic acid amplification assays (e.g., polymerase chain reaction assays), combined reverse transcription/nucleic acid amplification, nuclease protection (SI nuclease or RNAse protection assays), Serial Analysis Gene Expression (SAGE), next generation sequencing, gene expression microarray, as well as other methods.
The probe can be labelled by any of the many different methods known to those skilled in this art. The labels most commonly employed for these studies are radioactive elements, enzymes, chemicals that fluoresce when exposed to ultraviolet light, and others. A number of fluorescent materials are known and can be utilized as labels. These include, but are not limited to, fluorescein, rhodamine, auramine, Texas Red, AMCA blue and Lucifer Yellow. The radioactive label can be detected by any of the currently available counting procedures. Non-limiting examples of isotopes include ³H, ¹⁴C, ³²P, ³⁵S, ⁵¹Cr, ⁵⁷Co, ⁵⁸Co, 59Fe, ⁹⁰Y, ¹²⁵I, ¹³¹I, and ¹⁸⁶Re.
An antibody or fragment thereof that specifically binds a CXCL14 polypeptide, preferably the human CXCL14 polypeptide can be used to assay to measure circulating CXCL14 polypeptide. The term "antibody" relates to a monomeric or multimeric protein which comprises at least one polypeptide having the capacity for binding to a determined antigen and comprising all or part of the light or heavy chain variable region of an immunoglobulin molecule. The term antibody includes any type of known antibody, such as, for example, polyclonal antibodies, monoclonal antibodies and genetically engineered antibodies, such as chimeric antibodies, humanized antibodies, primatized antibodies, human antibodies and bispecific antibodies. The invention also comprises the use of fragments of the different types of antibodies mentioned above which substantially preserve the anti-angiogenic activity of the antibody. The term "antibody fragment" includes antibody fragments such as Fab, F(ab')2, Fab', single chain Fv fragments (scFv), diabodies and nanobodies.
The phrase "specifically bind(s)" or "bind(s) specifically" when referring to a peptide refers to a peptide molecule which has intermediate or high binding affinity, exclusively or predominately, to a target molecule. The phrase "specifically binds to" refers to a binding reaction that is determinative of the presence of a target protein in the presence of a heterogeneous population of proteins and other biologies. Thus, under designated assay conditions, the specified binding moieties bind preferentially to a particular target protein and do not bind in a significant amount to other components present in a test sample. Specific binding to a target protein under such conditions may require a binding moiety that is selected for its specificity for a particular target antigen. A variety of assay formats may be used to select ligands that are specifically reactive with a particular protein. For example, solid-phase ELISA immunoassays, immunoprecipitation, Biacore, and Western blot are used to identify peptides that specifically react with human or murine CXCL14. Typically a specific or selective reaction will be at least twice background signal or noise and more typically more than 10 times background. It is to be understood that that affinity interactions between and aptamer and an analyte or target or antibody and an analyte or target are a matter of degree. That is, the "specific binding affinity" of an aptamer or antibody for its target means that the aptamer or antibody binds to its target generally with a much higher degree of affinity than such aptamer or antibody may binds to other, non-target, components in a mixture or sample.
The term "Nucleic acid "means either DNA, RNA, single-stranded or double-stranded and any chemical modifications thereof, provided only that the modification does not interfere with amplification of selected nucleic acids. Such modifications include, but are not limited to, modifications at cytosine exocyclic amines, substitution of 5-bromo-uracil, backbone modifications, methylations, unusual base-pairing combinations and the like. The terms "nucleic acid," "oligonucleotide," and "polynucleotide" are used interchangeably to refer to a polymer of nucleotides of any length, and such nucleotides may include deoxyribonucleotides, ribonucleotides, and/or analogs or chemically modified deoxyribonucleotides or ribonucleotides. The terms "polynucleotide," "oligonucleotide," and "nucleic acid" include double- or single-stranded molecules as well as triple-helical molecules.

The term "Ligand" or "ligand of a given target" means a nuclei acid that binds another molecule (target). In a population of candidate nucleic acids, a ligand is one which binds with greater affinity than that of the bulk population. In a candidate mixture there can exist more than one ligand for a given target. The ligands can differ from one another in their binding affinities for the target molecule.

The present invention shows that PCOS associates with reduced levels of CXCL14, a chemokine recently proposed to be secreted preferentially by brown/beige adipose tissue (2). The restoration of normal levels of CXCL14 with SPIOMET, the capacity of pioglitazone to increase CXCL14 expression in pre-adipocytes and the capacity of spironolactone to increase the release of CXCL14 in adipocytes, respectively, indicated that SPIOMET treatment targets adipose tissue to improve the metabolic profile of PCOS patients. In addition, the normalization of central fat after SPIOMET treatment indicates that circulating CXCL14 relates to ectopic fat rather than to BMI *per se.*
High CXCL14 levels have been shown to improve insulin sensitivity in adipocytes *in vitro* (11) and in rodent models (2); however, not all reports agree in the anti-diabetic actions of CXCL14, and even deleterious pro-diabetogenic effects have been proposed *in vitro* (12) and *in vivo* (13). The present invention indicates that normalization of CXCL14 after SPIOMET associates with decreased insulin resistance. Pioglitazone increases insulin sensitivity as well as adipogenic differentiation, and also promotes the acquisition of a brown/beige phenotype in adipose cells (14). The induction of CXCL14 expression by pioglitazone is consistent with data in experimental models highlighting increased expression of CXCL14 with brown/beige adipogenic differentiation (2). The positive effects of spironolactone inducing CXCL14 in differentiated adipocytes are also consistent with previous reports indicating that spironolactone induces adipose tissue browning in rodents (15) and activates brown fat in humans (16). Recent reports disclosed impaired brown adipose tissue activity in PCOS subjects (6,7) and the capacity of brown fat activation to ameliorate PCOS in experimental models (8). Considering that CXC14 is preferentially released by brown adipocytes (2), it emerges that SPIOMET treatment drives a shift in adipose tissue plasticity to a more brown/beige phenotype resulting in enhanced CXCL14 release, potentially improving glucose homeostasis and possibly reducing diabetes risk. This sequence is especially relevant in young girls, where the brown/beige adipose tissue amount is particularly significant (17).

### EXAMPLES

### Example 1. Research Design & Methods

### Study population and design

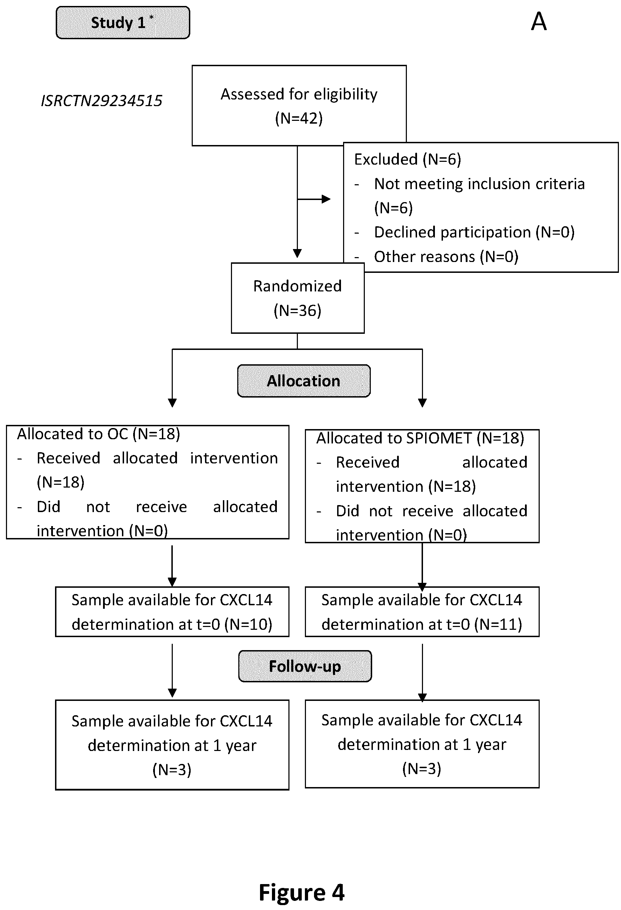

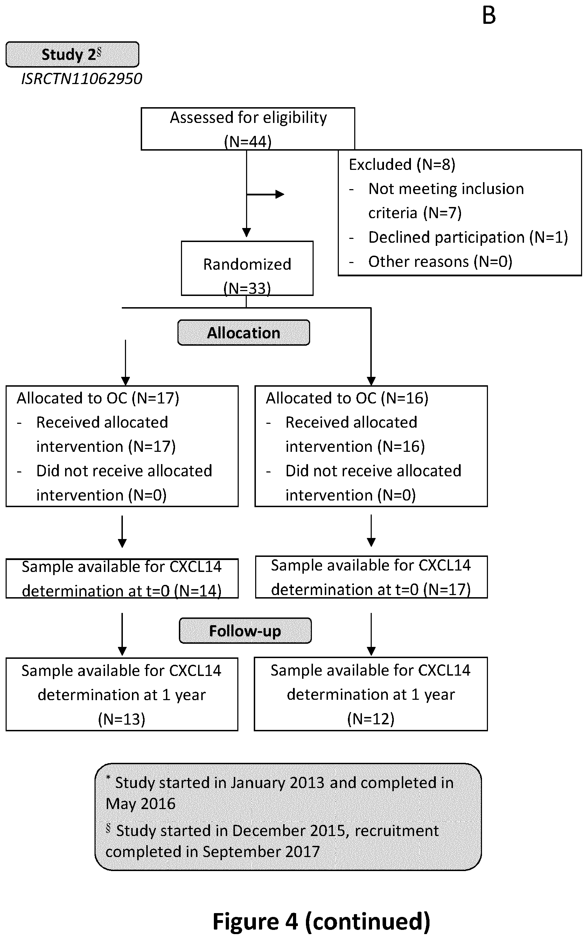

The study population consisted of 52 adolescent girls with PCOS [age, 15.6yr; body mass index (BMI), 24.3 kg/m²] who were enrolled into two randomized, open-label, controlled trials (with identical design) exploring the effects of OC *versus* SPIOMET treatment for one year, with post-treatment ovulation rate as primary outcome. The results of the first trial (ISRCTN29234515) have been already published (5); the second trial (ISCRCTN11062950) will be completed in 2019. Twenty-one out of the 52 girls belonged to the first study and 31 to the second trial; 31 of the 52 had available samples for CXCL14 assessment at 0 and 12 months (***Figure 4***). Both trials were performed at Sant Joan de Déu University Hospital, Barcelona, Spain. Inclusion and exclusion criteria have been described previously (5). OC treatment consisted of 20 µg of ethinylestradiol plus 100 mg of levonorgestrel for 21/28 days and placebo for 7/28 days; SPIOMET is a low-dose combination of spironolactone 50 mg/d, pioglitazone 7.5 mg/d, and metformin 850 mg/d. Twenty-one age-matched healthy girls recruited in nearby schools served as controls. All had regular menstrual cycles and none was hirsute or taking medications.

### Clinical, Endocrine-Metabolic, and Imaging Assessments

Birth weight and BMI (and their Z-scores) were retrieved, and endocrine-metabolic variables were assessed in the early morning, in the follicular phase (days 3-7) of the cycle or after 2 months of amenorrhea (5). Serum glucose, insulin, homeostasis model assessment (HOMA)-insulin resistance, lipids, ultrasensitive C-Reactive protein, sex hormone-binding globulin (SHBG), androgens and high-molecular-weight (HMW)-adiponectin were assessed as reported (5). CXCL14 levels in serum and cell culture medium were determined using a specific ELISA kit (RayBiotech) (2), whose sensitivity was 0.7 ng/ml, and inter-assay and intra-assay CV less than 12%. Body composition was assessed by dual X-ray absorptiometry with a Lunar Prodigy and Lunar software (version 3.4/3.5, Lunar Corp, Madison, Wisconsin); abdominal (subcutaneous and visceral) and hepatic fat were assessed by magnetic resonance imaging (MRI) using a multiple-slice MRI 1.5 Tesla scan (Signa LX Echo Speed Plus Excite, General Electric, Milwaukee, Wisconsin) (5). Central fat was arbitrarily defined as the sum of visceral fat (in cm²) and hepatic fat (in %).

### Studies in human adipocytes in culture

The effects of pioglitazone, spironolactone and metformin on adipocytes were studied in human SGBS cells, a cell model of human beige (brown-like) adipogenesis (9,10). SGBS pre-adipocytes were maintained in DMEM/F12, 10% FBS. Adipogenic differentiation was initiated by incubating confluent cell cultures for 4 days in serum-free medium plus 20 nM insulin, 0.2 nM triiodothyronine, 100 nM cortisol, 25 nM dexamethasone, 500 µM 3-isobutyl-methyl-xanthine, 2 µM rosiglitazone. Subsequently, the cells were switched to DMEM/F12, 20 nM insulin, 0.2 nM triiodothyronine and 100 nM cortisol and maintained for up to 10 days, when more than 90% cells have acquired differentiated adipocyte morphology. Two experimental designs were followed: a) cells were treated with the medications across their differentiation process, in the absence of rosiglitazone and maintaining the drugs throughout the 10 days of differentiation, b) adipocytes were treated with the drugs acutely (24h), once the cells have been differentiated. Controls included a 1:1 methanol/DMSO mixture (drug solvents) at ≤ 1/1000 concentration. Cell culture reagents and drugs were from Sigma. The cell culture medium was collected and concentrated 1:5 prior to measurement of CXCL14 levels. RNA was extracted from cells using an affinity column-based method (Machery-Nagel). Real-time quantitative reverse transcription polymerase chain reactions (qRT-PCR) were performed using 0.5 µg RNA and employing Taqman reagents and probes (Life Technologies), according to supplier indications. PCR was conducted in an ABI/Prism-7700 Sequence Detector System. The following TaqMan probes were used: CXCL14, Hs01557413; UCP1, Hs00222453; FABP4, Hs00609791 and RPLP0 mRNA, Hs99999902. Each sample was run in duplicate and the mean value was used to calculate the relative amount of individual mRNAs. Each mean value was normalized to that of the RPLP0 mRNA using the comparative (2-ΔCT) method.

### Statistical analysis and ethics

Statistical analyses were performed with SPSS 23.0 (SPSS, Chicago, IL). Baseline differences in CXCL14 concentrations between patients and controls were tested with unpaired *t* test, adjusting for age and BMI. Longitudinal changes between groups were compared by repeated-measures general linear model. Differences in longitudinal changes between groups were tested by the interaction term among between- and within-subject effects. Associations were sought by Pearson correlation analysis. P < 0.05 was considered statistically significant. Results are expressed as mean ± SEM.

### Example 2 Serum CXCL14 levels are reduced in adolescent girls with PCOS

At baseline, PCOS girls showed lower SHGB levels, higher total testosterone concentrations and free androgen index (FAI), increased central and hepatic fat, and a trend towards higher insulin and lower HMW-adiponectin levels versus controls (***Table 1***).

**Table 1. Study variables in healthy control girls (N= 21) and in girls with polycystic ovary syndrome (PCOS, N= 52).**

| | **Controls (N= 21)** | **PCOS (N= 52)** |
|---|---|---|
| **Auxology** | | |
| Age (years) | 16.0 ± 0.3 | 15.6 ± 0.2 |
| Birth weight Z-score | 0.2 ± 0.2 | -0.6 ± 0.1* |
| BMI (kq/m2) | 21.5 ± 0.6 | 24.3 ± 0.6** |
| BMI Z-score | 0.1 ± 0.2 | 0.9 ± 0.2* |
| Δ Z-score birth weight - BMI | -0.2 ± 0.2 | 1.4 ± 0.2* |

| **Endocrine-Metabolic variables** | | |
|---|---|---|
| Testosterone (nmol/ L) | 1.0 ± 0.1 | 1.3 ± 0.1* |
| SHBG (nmol/ L) † | 61.1 ± 6.8 | 30.9 ± 1.7*** |
| FAI † | 1.8 ± 0.2 | 4.8 ± 0.4*** |
| Glucose (mmol/ L) | 4.9 ± 0.1 | 4.6 ± 0.1** |
| Fasting insulin (pmol/ L) | 50.2 ± 6.1 | 74.5 ± 5.9 |
| HOMA-IR | 1.6 ± 0.2 | 2.3 ± 0.2 |
| HDL-cholesterol (nmol/ L) | 1.4 ± 0.0 | 1.3 ± 0.0 |
| LDL-cholesterol (nmol/ L) | 2.2 ± 0.1 | 2.3 ± 0.1 |
| Triglycerides (nmol/ L) | 0.6 ± 0.0 | 0.7 ± 0.0 |
| HMW adiponectin (mg/ L) | 9.1 ± 1.4 | 6.3 ± 0.7 |
| usCRP (mg/ L) † | 0.6 ± 0.1 | 1.5 ± 0.3 |

| **Body composition (DXA) ^{‡}** | | |
|---|---|---|
| Bone mineral density (g/ cm2) | - | 1.18 ± 0.01 |
| Lean mass (kg) | - | 35.9 ± 0.7 |
| Fat mass (kg) | - | 22.9 ± 1.2 |
| Abdominal fat (kg) | - | 6.1 ± 0.3 |

| **Abdominal fat partitioning (MRI) ^{†}** | | |
|---|---|---|
| Subcutaneous fat (cm2) | 95 ± 13 | 184 ± 17 |
| Visceral fat (cm2) | 27 ± 2 | 42 ± 3 |
| Hepatic fat (%) | 10 ± 1 | 18 ± 1*** |
| Central (hepato-visceral) fat | 37 ± 3 | 60 ± 3** |

| | | |
|---|---|---|
| Values are mean ± SEM. BMI, body mass index; SHBG, sex hormone-binding globulin; FAI, free androgen index; HOMA-IR, homeostasis model assessment insulin resistance; HDL, high-density lipoprotein; LDL, low-density lipoprotein; HMW, high molecular weight; usCRP, ultra-sensitive C-reactive protein; DXA, dual X-ray absorptiometry; MRI, magnetic resonance imaging. ^{†}SHBG, FAI, usCRP and abdominal fat partitioning assessments were performed in 15 out of 21 healthy adolescent girls. ^{‡} Indicative DXA values in healthy adolescents, matched for age and height (n=41): lean mass 35.1 ± 1.0 kg; fat mass 17.6 ± 1.4 kg (Ibáñez L, et al. J Adolesc Health 2017; 61: 446-453). P values are adjusted for age and BMI. * P≤0.05; ** P≤0.01; and *** P≤0.001 between controls and girls with PCOS. | | |

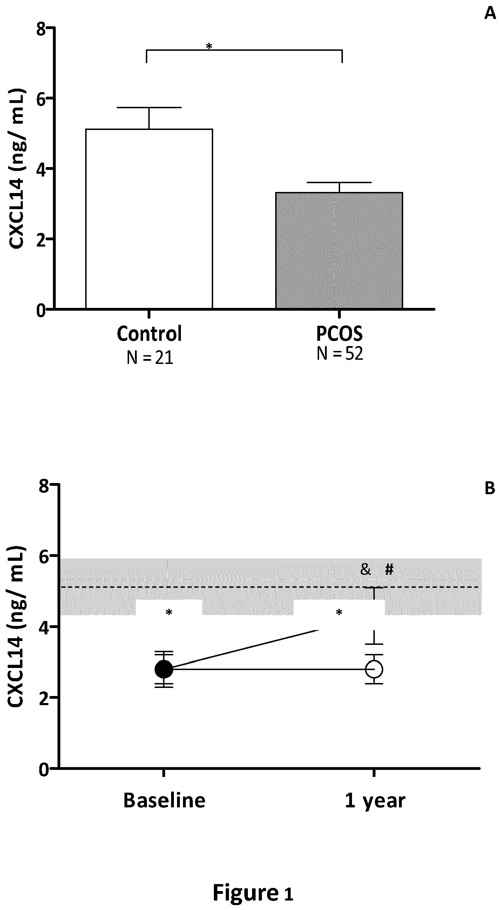

None of the girls were obese (BMI ≥30 Kg/m²); however, mean BMI was higher in girls with PCOS as compared to control girls (p=0.01). Fasting glucose levels were within the normal range in both subgroups, and were marginally higher in control girls (***Table 1***). Serum CXCL14 concentrations were reduced in girls with PCOS to close to two-thirds the normal levels (***Figure 1A***), did not correlate with markers of adiposity or glucose homeostasis, and were positively associated with HMW-adiponectin levels (***Table 2***).

**Table 2. Correlations between circulating baseline CXCL14 concentrations and selected variables in the study population [N= 21 controls and N= 52 girls with Polycystic Ovary Syndrome (PCOS].**

| All subjects (N= 73) | | |
|---|---|---|
| | CXCL14 (ng/ mL) | |
| | R | P |
| **Auxological variables** | | |
| Aqe (yr) | -0.130 | 0.273 |
| Birth weight Z-score | 0.035 | 0.768 |
| BMI (kg/m2) | -0.157 | 0.185 |
| BMI Z-score | -0.137 | 0.247 |
| Δ Z-score birth weight - BMI | -0.124 | 0.299 |

| **Endocrine-Metabolic variables** | | |
|---|---|---|
| Testosterone (nmol/ L) | -0.023 | 0.854 |
| SHBG (nmol/ L) ^{†} | 0.039 | 0.756 |
| FAI ^{†} | -0.099 | 0.441 |
| Glucose (mmol/ L) | 0.224 | 0.058 |
| Fasting insulin (pmol/ L) | -0.102 | 0.392 |
| HOMA-IR | -0.083 | 0.485 |
| HDL-cholesterol (nmol/ L) | 0.039 | 0.740 |
| LDL-cholesterol (nmol/ L) | 0.058 | 0.627 |
| Triglycerides (nmol/ L) | -0.125 | 0.290 |
| HMW adiponectin (mg/ L) | 0.275 | 0.023 |
| usCRP (mg/ L) ^{†} | -0.195 | 0.115 |

| **Abdominal fat partitioning (MRI) ^{†}** | | |
|---|---|---|
| Subcutaneous fat (cm2) | -0.158 | 0.202 |
| Visceral fat (cm2) | 0.025 | 0.844 |
| Hepatic fat (%) | -0.238 | 0.052 |
| Central (hepato-visceral) fat | -0.049 | 0.692 |

| | | |
|---|---|---|
| CXCL14, C-X-C motif chemokine ligand-14, BMI, body mass index; SHBG, sex hormone-binding globulin; FAI, free androgen index; HOMA-IR, homeostasis model assessment insulin resistance; HDL, high-density lipoprotein; LDL, low-density lipoprotein; HMW, high molecular weight; usCRP, ultra-sensitive C-reactive protein; DXA, dual X-ray absorptiometry; MRI, magnetic resonance imaging. ^{†} SHBG, FAI, usCRP and abdominal fat partitioning assessments were performed in 67 subjects (15 control girls and 52 girls with PCOS). Results are Pearson correlation coefficients and P values. | | |

### Example 3 SPIOMET, but not OC, normalizes serum CXCL14 levels in adolescent girls with PCOS

SPIOMET treatment exerted more benefits than OC on endocrine-metabolic and imaging markers, including on fasting insulin, HOMA-IR, HMW-adiponectin, usCRP and hepato-visceral (central) fat, consistent with previous observations (5) (***Table 3***).

**Table 3. Auxological, endocrine-metabolic, body composition (by DXA), and abdominal fat partitioning (by MRI) assessments in adolescent girls with PCOS who were randomized to receive oral contraception (OC, N=16) or a low-dose combination of Spironolactone (50mg/ d), Pioglitazone (7.5 mg/d) and Metformin (850 mg/d) (SPIOMET, N=15) for 1 year.**

| | OC ^{†} (N= 16) | | | SPIOMET (N= 15) | | |
|---|---|---|---|---|---|---|
| | Start ^{‡} | 1 year | Δ 0-1 year | Start ^{‡} | 1 yea r | Δ 0-1 year |
| **Auxology** | | | | | | |
| Age (years) | 15.8± 0.3 | 16.9± 0.3 | - | 15.5± 0.4 | 16.6 ± 0.4 | - |
| Birth weight Z-score | -0.5± 0.2 | - | - | -0.2± 0.3 | - | - |
| BMI (kg/m2) | 25 ± 1 | 26 ± 1 | 1.2 ± 0.3 | 25 ± 1 | 25 ± 1 | 0.0 ± 0.6 |
| BMI Z-score | 0.9 ± 0.3 | 1.3 ± 0.4 | 0.4 ± 0.1 | 1.1 ± 0.4 | 1.1 ± 0.4 | -0.1 ± 0.2 |
| Δ Z-score birth weight - BMI | 1.4 ± 0.3 | - | - | 1.3 ± 0.5 | - | - |

| **Endocrine-Metabolic Variables** | | | | | | |
|---|---|---|---|---|---|---|
| Testosterone (nmol/ L) | 1.3 ± 0.2 | 0.9 ± 0.1 | -0.4 ± 0.2 | 1.3 ± 0.1 | 1.0 ± 0.1 b | -0.3 ± 0.1 |
| SHBG (nmol/ L) | 34 ± 3 | 57 ± 6 c | 23 ± 5 | 31 ± 4 | 29±3 *** | -2 ± 2 £ |
| FAI | 4.7 ± 0.6 | 2.6 ± 1.1 | -2.1± 1.3 | 5.0 ± 0.8 | 3.6 ± 0.6 | -1.4 ± 0.8 |
| Glucose (mmol/ L) | 4.5 ± 0.1 | 4.5 ± 0.1 | 0.0 ± 0.1 | 4.5 ± 0.1 | 4.2±0.1 * | -0.3 ± 0.1 |
| Fasting insulin (pmol/ L) | 78 ± 10 | 91 ± 11 | 13 ± 8 | 69 ± 7 | 49 ± 6 **b | -20 ± 9 § |
| HOMA-IR | 2.3 ± 0.3 | 2.6 ± 0.3 | 0.3 ± 0.3 | 2.0 ± 0.2 | 1.4±0.2**b | -0.6±0.3§ |
| HDL-cholesterol (nmol/ L) | 1.3 ± 0.1 | 1.3 ± 0.1 | 0.0 ± 0.0 | 1.3 ± 0.0 | 1.4 ± 0.1 | 0.1 ± 0.0 |
| LDL-cholesterol (nmol/ L) | 2.3 ± 0.1 | 2.6 ± 0.2 a | 0.3 ± 0.1 | 2.0 ± 0.1 | 2.0±0.1** | 0.0 ± 0.1 |
| Triglycerides (nmol/ L) | 0.7 ± 0.1 | 0.7 ± 0.1 | 0.0 ± 0.0 | 0.7 ± 0.1 | 0.7 ± 0.1 | 0.0 ± 0.0 |
| HMW adiponectin (mg/ L) | 5 ± 1 | 5 ± 1 | 0 ± 1 | 5 ± 1 | 9 ± 2 a | 4 ± 2 § |
| usCRP (mg/L) | 1.0 ± 0.2 | 2.1 ± 0.5 | 1.1 ± 0.4 | 1.7 ± 0.4 | 1.1 ± 0.3 | -0.6±0.4¶ |

| **Body composition (DXA)** | | | | | | |
|---|---|---|---|---|---|---|
| Bone mineral density (g/cm2) | 1.19 ± 0.03 | 1.19 ± 0.03 | 0.00 ± 0.01 | 1.21 ± 0.03 | 1.20 ± 0.03 | -0.01 ± 0.01 |
| Lean mass (kg) | 37 ± 1 | 38 ± 2 a | 1 ± 1 | 36 ± 1 | 36 ± 1 ** | 0 ± 1 |
| Fat mass (kg) | 24 ± 2 | 26 ± 2 c | 2 ± 1 | 25 ± 3 | 25 ± 2 | 0 ± 1 |
| Abdominal fat (kg) | 6.2 ± 0.5 | 6.7 ±0.6 a | 0.5 ± 0.2 | 6.5 ± 0.6 | 6.2±0.6** | -0.3±0.3§ |

| **Abdominal fat partitioning (MRI)** | | | | | | |
|---|---|---|---|---|---|---|
| Subcutaneous fat (cm2) | 192 ± 28 | 217±29 b | 25 ± 8 | 188 ± 35 | 183 ± 33 | -5 ± 7 § |
| Visceral fat (cm2) | 41 ± 5 | 45 ± 6 | 4 ± 4 | 40 ± 4 | 38 ± 4 | -2 ± 2 |
| Hepatic fat (%) | 17 ± 1 | 19 ± 2 | 2 ± 2 | 20 ± 1 | 10± 1 ***c | -10 ± 1 £ |
| Central (hepato-visceral) fat | 58 ± 6 | 64 ± 7 | 6 ± 5 | 60 ± 5 | 48 ± 5 **c | -12 ± 3 ¶ |

| | | | | | | |
|---|---|---|---|---|---|---|
| Values are mean ± SEM. BMI, body mass index; SHBG, sex hormone-binding globulin; FAI, free androgen index; HOMA-IR, homeostasis model assessment insulin resistance; HDL, high-density lipoprotein; LDL, low-density lipoprotein; HMW, high molecular weight; usCRP, ultra-sensitive C-reactive protein; DXA, dual X-ray absorptiometry; MRI, magnetic resonance imaging. ^{†} OC: Ethinylestradiol 20 µg plus Levonorgestrel 100 mg ^{‡} no significant differences between randomized subgroups at start. * P ≤ 0.05, ** P ≤ 0.01 and *** P ≤ 0.001 for differences between subgroups at 1 year. § P ≤ 0.05, ¶ P ≤ 0.01 and £ P ≤ 0.001 for differences between subgroup changes (Δ) 0-1 year. a P ≤ 0.05, b P ≤ 0.01 and c P ≤ 0.001 for differences within-subgroup changes from start. P values for differences between subgroups are adjusted for age and BMI. | | | | | | |

Serum CXCL14 levels increased significantly after SPIOMET, reaching levels similar to those in controls, and remained significantly decreased after OC, as compared to controls and to SPIOMET-treated girls (***Figure 1B***)*.* The increase in CXCL14 levels in the SPIOMET-treated patients correlated significantly with the extent of reduction in fasting insulin and HOMA-IR (***Table 4***).

**Table 4. Correlations between 0-1 year changes (Δ) in serum CXCL14 levels and those in clinical, endocrine-metabolic, body composition, and abdominal fat partitioning variables in girls with Polycystic Ovary Syndrome (PCOS, N=15) who received a low-dose combination of Spironolactone (50 mg/d), Pioglitazone (7.5 mg/d) and Metformin (850 mg/d) (SPIOMET) for 1 year.**

| **SPIOMET (N= 15)** | | |
|---|---|---|
| | **Δ CXCL14 (ng/ mL)** | |
| | R | P |
| **Auxology** | | |
| Δ BMI (kg/m2) | -0.108 | 0.702 |
| Δ BMI Z-score | -0.108 | 0.703 |

| **Endocrine-Metabolic variables** | | |
|---|---|---|
| Δ Testosterone (nmol/ L) | 0.293 | 0.331 |
| Δ SHBG (nmol/ L) | -0.144 | 0.608 |
| Δ FAI | 0.017 | 0.955 |
| Δ Glucose (mmol/ L) | 0.511 | 0.051 |
| Δ Fasting insulin (pmol/ L) | 0.588 | 0.021 |
| Δ HOMA-IR | 0.630 | 0.012 |
| Δ HDL-cholesterol (nmol/ L) | -0.198 | 0.498 |
| Δ LDL-cholesterol (nmol/ L) | -0.446 | 0.096 |
| Δ Triglycerides (nmol/ L) | -0.045 | 0.874 |
| Δ HMW adiponectin (mg/ L) | 0.037 | 0.903 |
| Δ usCRP (mg/ L) | -0.009 | 0.980 |

| Body composition (DXA) | | |
|---|---|---|
| Δ Bone mineral density (g/ cm2) | -0.205 | 0.463 |
| Δ Lean mass (kg) | -0.176 | 0.531 |
| Δ Fat mass (kg) | -0.036 | 0.900 |
| Δ Abdominal fat (kg) | -0.243 | 0.384 |

| Abdominal fat partitioning (MRI) | | |
|---|---|---|
| Δ Subcutaneous fat (cm2) | -0.111 | 0.693 |
| Δ Visceral fat (cm2) | -0.187 | 0.504 |
| Δ Hepatic fat (%) | 0.226 | 0.417 |
| Δ Central (hepato-visceral) fat | -0.083 | 0.769 |

CXCL14, C-X-C motif chemokine ligand-14, BMI, body mass index; SHBG, sex hormone-binding globulin; FAI, free androgen index; HOMA-IR, homeostasis model assessment insulin resistance; HDL, high-density lipoprotein; LDL, low-density lipoprotein; HMW, high molecular weight; usCRP, ultra-sensitive C-reactive protein; DXA, dual X-ray absorptiometry; MRI, magnetic resonance imaging.

Results are Pearson correlation coefficients and P values.

### Example 4 Effects of the SPIOMET components on CXCL14 expression and release by human adipocytes

The effects of the three components of SPIOMET on human adipocyte differentiation were assessed individually and in combination (***Figure 2***). Pioglitazone had a dose-response dramatic effect inducing CXCL14 mRNA expression, whereas neither spironolactone nor metformin did. No concentration higher than 10 µM could be tested for spironolactone, because of cell toxicity upon pre-adipocytes. Indeed, pioglitazone at the lowest concentration tested (0.1 µM) already caused a close to 10-fold induction of CXCL14 mRNA expression (***Figure 2A***). This effect paralleled a strong positive effect of pioglitazone on adipogenic differentiation, as evidenced by lipid droplet accumulation (***Figure 2C***) and expression of gene markers of general adipogenic [fatty acid binding protein-4 (FABP4)] and beige adipogenic [uncoupling protein-1 (UCP1)] differentiation (***Figure 2D***).

Addition of metformin to pioglitazone did not modify the extent of pioglitazone induction of CXCL14 mRNA expression (***Figure 2B***)*.* However, the addition of spironolactone reduced significantly the effects of pioglitazone on CXCL14 mRNA expression. The induction of CXCL14 mRNA expression caused by the three drugs in combination was similar to that elicited by pioglitazone plus spironolactone. These data also paralleled the observations on adipogenic differentiation after combined treatments, according to cell morphology and expression of FABP4 and UCP1 (***Figures 2C and 2D***). CXCL14 protein was only detectable in the cell culture medium of differentiating cells under pioglitazone treatment, either alone (0.185 ng/mL) or in combination with spironolactone and metformin (0.179 ng/mL).

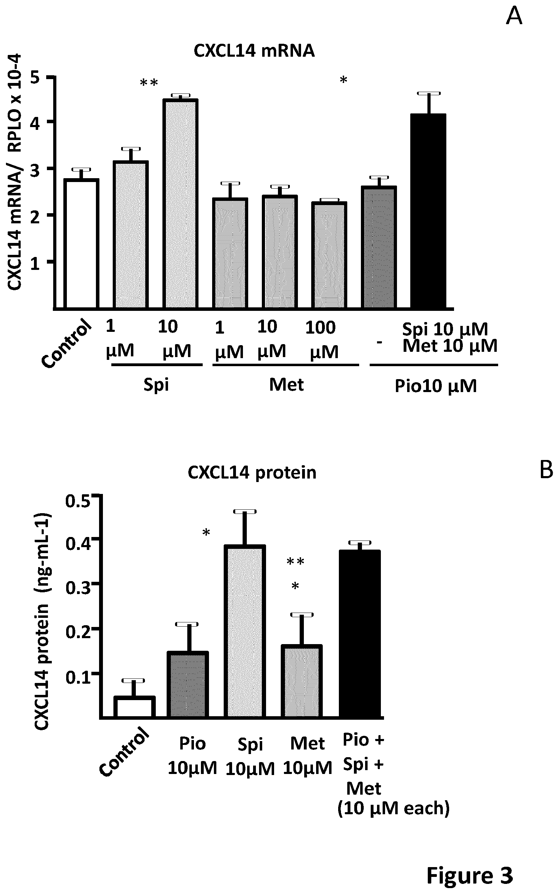

Neither pioglitazone nor metformin alone had any effect on CXCL14 mRNA expression on already differentiated human adipocytes (***Figure 3A***). However, 10 µM of spironolactone caused a significant induction of CXCL14 mRNA expression (***Figure 3A***). The combination of three components (with spironolactone dosed at 10 µM), induced CXCL14 mRNA to the same extent as treatment with spironolactone alone. Only spironolactone, either alone or in combination with pioglitazone plus metformin, elicited a significant increase (close to 8-fold) of the CXCL14 protein levels released by adipocytes to the cell culture medium (***Figure 3B***)*.*

### References

1. Cereijo R, et al. Ann Med 2015; 47:169-177.
2. Cereijo R, et al. Cell Metab 2018; 28:750-763.
3. de Zegher F et al. Trends Endocrinol Metab 2018; 29:815-818.
4. Ibáñez L & de Zegher F. Pediatr Obes 10.1111/ijpo.2019.9999.issue-9999.
5. Ibáñez L et al. J Adolesc Health 2017; 61:446-453.
6. Shorakae S. et al. Clin Endocrinol (Oxf) 2019; 90:425-432.
7. Oliveira FR. et al. Eur J Endocrinol 2019; pii: EJE-19-0505.R1.
8. Yuan X. et al. Proc Natl Acad Sci USA 2016; 113:2708-2713.
9. Wabitsch M. et al.. Int J Obes Relat Metab Disord 2001; 25:8-15.
10. Yeo CR. et al.. Sci Rep 2017; 7:4031.
11. Takahashi M. et al. Biochem Biophys Res Commun. 2007;364:1037-1042.
12. Atanes P. et al. Cell Physiol Biochem. 2019;52(4):879-892.
13. Nara N. et al.. J Biol Chem. 2007; 282:30794-30803.
14. Foellmi-Adams LA. et al. Biochem Pharmacol 1996; 52:693-701.
15. Armani A. et al. FASEB J 2014; 28:3745-3757.
16. Thuzar M. et al. Diabetes Obes Metab 2019; 21:509-516.
17. Gilsanz V. et al. Pediatr Res 2013; 73:3-9.
18. Ballester MR. et al. 2nd International Conference on Medicinal Chemistry & Drug Design and 2nd International Conference on Bio Equivalence & Bio Availability; Barcelona, 2019 (pp 53 Abstract book);
19. Corbould A. Horm Metab Res 2007; 39:915-918

## Claims

1. In vitro use of reagents for measuring CXCL14 in a serum sample, in the diagnosis of PCOS in an adolescent girl.

2. The use according to claim 1, wherein the adolescent girl is non-obese.

3. In vitro use of reagents for measuring CXCL14 in serum in monitoring the efficacy of a PCOS treatment in an adolescent girl.

4. The use according to claim 3, wherein the PCOS treatment is a treatment with with spironolactone, pioglitazone and metformin (SPIOMET).

5. The use according to claim 3 or 4, wherein the adolescent girl is non-obese.

6. The use according to any one of claims 3 to 5, wherein the monitoring identifies responders or non responders to said treatment.

7. The use according to anyone of claims 3 to 8, wherein an increase of the concentation of CXCL14, compared to the concentation prior to the treatment is indicative of the patient being a responder.

8. The use according to any one of claims 1 to 7 wherein the reagents are oligonucleotides for the detection of CXCL14 mRNA.

9. The use according to any one of claims 1 to 7, wherein the reagents are binding agents specifically binding to CXCL14 protein.

10. In vitro method of diagnosing PCOS in an adolescent girl, comprising the steps of:
determining in a serum sample of said adolescent girl, the concentration of CXCL14,
comparing the determined concentation with a reference value of a healthy adolescent girl,
determining from said comparison, whether said adolescent girl is a PCOS patient.

11. The method according to claim 10, wherein a low concentation of CXCL14 in the sample compared to said reference value is indicative for said adolescent girl being a PCOS patient.

12. In vitro method for monitoring the efficacy of a PCOS treatment in an adolescent girl, comprising the steps of:
determining in a serum sample of said adolescent girl prior to or at the onset of said treatment, a first concentration of CXCL14,
determining during the treatment in a serum sample of said adolescent girl, a second concentration of CXCL14,
comparing the first and the second concentratiob, wherein an increase of the concentration, compared to the first concentration is indicative of the adolescent girl being a reponder to said treatment.

13. The method according to claim 12, wherein the treatment is a treatment with spironolactone, pioglitazone and metformin (SPIOMET).

14. The method according to claim 12 or 13, wherein the step of determining the second concentration is performed between 1 and 12 months after the onset of said treatment.
